Europäisches Patentamt

European Patent Office

Office européen des brevets

(19)

(11) Veröffentlichungsnummer: **0 082 513**
**B1**

(12) **EUROPÄISCHE PATENTSCHRIFT**

(45) Veröffentlichungstag der Patentschrift:
06.03.85

(51) Int. Cl.⁴: **C 07 C 9/04**, C 07 C 7/148,
C 10 G 27/12, E 21 B 41/02

(21) Anmeldenummer: 82111800.7

(22) Anmeldetag: 20.12.82

(54) Verfahren zur Beseitigung von H2S aus Erdgas, Erdöl und deren Gemischen.

(30) Priorität: 23.12.81 DE 3151133

(43) Veröffentlichungstag der Anmeldung:
29.06.83 Patentblatt 83/26

(45) Bekanntmachung des Hinweises auf die Patenterteilung:
06.03.85 Patentblatt 85/10

(84) Benannte Vertragsstaaten:
BE DE GB NL SE

(56) Entgegenhaltungen:
FR - A - 2 119 506
FR - A - 2 271 862
GB - A - 424 616

OIL & GAS JOURNAL, Band 80, Nr. 36, 6. September
1982, Seiten 118-123, Tulse, Oklahoma, USA R.A.
BROWN et al.: "Hydrogen peroxide reduces sulfide
corrosion"

(73) Patentinhaber: Peroxid-Chemie GmbH,
Dr.-Gustav-Adolph-Strasse 2, D-8023 Höllriegelskreuth
bei München (DE)

(72) Erfinder: Schwarzer, Hans, Dr., Stobäusstrasse 28,
D-8190 Wolfratshausen (DE)
Erfinder: Meisel, Werner, Dipl.-Ing., Steiner Ring 99,
D-8192 Geretsried 2 (DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)

## Beschreibung

Die Erfindung betrifft die Beseitigung von $H_2S$ aus Erdgas, Erdöl und deren Gemischen.

Es ist bekannt, daß Erdöl oder Erdgas vielfach erhebliche Mengen an $H_2S$ enthalten. Da $H_2S$ sowohl die Vorrichtungen, mit denen das Erdgas bzw. Erdöl in Berührung kommt, stark korrodiert und außerdem sehr giftig ist, muß es entfernt werden. Es ist bereits bekannt, $H_2S$ aus Naturgas mittels Essigsäure und $H_2O_2$ in Gegenwart eines Katalysators zu entfernen. Es ist anzunehmen, daß unter den angegebenen Bedingungen tatsächlich Peressigsäure als wirksames Reagenz vorliegt, da sie sich aus Essigsäure und $H_2O_2$ bildet. Ebenso ist es bekannt, auch andere organische Persäuren zur $H_2S$-Entfernung aus gasförmigen Kohlenwasserstoffen zu verwenden. Eine vollständige Entfernung gelang dabei jedoch nicht. Weiter ist es bekannt, aus geothermischem Wasserdampf den $H_2S$-Gehalt durch Einspritzen von $H_2O_2$ zusammen mit einem Alkali- oder Erdalkalihydroxyd herabzusetzen. Eine vollständige Beseitigung des $H_2S$ gelang hierdurch ebenfalls nicht. Aus der EP-A 29 472 ist es weiter bekannt, flüssige Kohlenwasserstoffe mit einer wäßrigen Lösung von $H_2O_2$ und einem metallionischen Katalysator, wie Ferrichlorid und Cuprichlorid zu behandeln, wobei auch Schwefelverbindungen beseitigt werden sollen.

Demgegenüber liegt der Erfindung die Aufgabe zugrunde, eine einfache Entfernung von $H_2S$ aus Erdgas und Erdöl bzw. deren Gemischen zu schaffen, welches weder Katalysatoren, noch organische Zusätze erfordert, eine vollständige Beseitigung des $H_2S$ aus der hydrophoben Gas- oder Flüssigkeitsphase erlaubt und unter Bedingungen durchführbar ist, welche bei der Förderung von Erdgas bzw. Erdöl häufig ohnedies auftreten.

Diese Aufgabe wird erfindungsgemäß gelöst durch ein Verfahren zur Beseitigung von $H_2S$ aus Erdgas, Erdöl und deren Gemischen, welches dadurch gekennzeichnet ist, daß man das aus der Erde geförderte, wasserhaltige Erdgas, Erdöl oder deren Gemisch bei einem Überdruck von mindestens 90 kPa und gegebenenfalls erhöhter Temperatur mit einer wäßrigen $H_2O_2$-Lösung versetzt und in einem Reaktor unter Aufrechterhaltung des erhöhten Drucks unter Mischungsbedingungen mindestens so lange reagieren läßt, bis in der Erdgasphase bzw. Erdölphase die gewünschte Herabsetzung des $H_2S$-Gehaltes eingetreten ist.

Überraschenderweise wurde gefunden, daß es unter den erfindungsgemäßen Bedingungen ohne Zusatz irgendwelcher Katalysatoren oder organischer Peroxide bzw. solche liefernden Verbindungen möglich ist, unerwünschtes $H_2S$ alleine mit $H_2O_2$ vollständig aus der organischen Phase zu entfernen. Dies konnte nicht erwartet werden, da ein Mehrphasensystem vorliegt und das $H_2S$ aus der hydrophoben organischen Phase mit einem in der wäßrigen Phase befindlichen Reagenz beseitigt wird und sogar in der Wasser-

dampfphase durch Zugabe von $H_2O_2$ in Kombination mit Alkalihydroxid und katalytisch wirkenden Metallionen bestenfalls eine 98%ige Entfernung von $H_2S$ gelang, obwohl es sich hierbei um ein einphasiges wäßriges System handelt.

Der Überdruck für die Umsetzung sollte zweckmäßig mindestens 90 kPa betragen, obwohl auch etwas niedrige Überdrücke unter Umständen noch anwendbar sind. Bevorzugt wird ein Überdruck von etwa 500 bis etwa 2000 kPa angewendet. Es ist daher möglich, bei Gas oder Öl, die unter Überdruck gefördert werden, die $H_2O_2$-Lösung direkt in die Druckleitung zu injizieren.

Die Temperatur kann beim erfindungsgemäßen Verfahren in weiten Bereichen variiert werden. Zweckmäßig sollte jedoch eine zu hohe Temperatur, bei der eine Selbstzersetzung des $H_2O_2$ begünstigt wird, vermieden werden. Zweckmäßig sind im allgemeinen Temperaturen zwischen 0 und 60°C, vorzugsweise zwischen 20 und 40°C.

Als Reaktoren kommen die hierfür bekannten Vorrichtungstypen in Betracht. Bevorzugt werden Röhrenreaktoren, da sich hier durch die Strömungsgeschwindigkeit und die lichte Weite des Rohres die erforderliche Vermischung der Phasen sowie die Reaktionsdauer (Verweilzeit) über die Strömungsgeschwindigkeit leicht regeln lassen. Beispielsweise kann als Röhrenreaktor eine Druckrohrstrecke verwendet werden, an derem Beginn die wäßrige $H_2O_2$-Lösung eingespritzt und an derem Ende der Restgehalt an $H_2S$ gemessen und, in Abhängigkeit vom Meßwert, die Einspritzmenge an $H_2O_2$ geregelt wird.

Anstelle eines Röhrenreaktors können jedoch auch andere Reaktortypen verwendet werden die es gestatten, durch geeignete Mischvorrichtungen eine gute Vermischung der hydrophoben Phase mit der wäßrigen Phase zu erzielen und den Druck für die erforderliche Zeitdauer aufrechtzuerhalten. Die Reaktionsdauer hängt normalerweise vom angewendeten Druck, der Temperatur und der Wirksamkeit der Vermischung ab. Im bevorzugten Druck- und Temperaturbereich und bei Verwendung eines Röhrenreaktors läßt sich in der Regel eine vollständige $H_2S$-Entfernung bei einer Einwirkungsdauer zwischen etwa 5 und etwa 20 Minuten erzielen.

Die zugeführte $H_2O_2$-Menge wird zweckmäßig so bemessen, daß sie vollständig abreagieren kann, d. h. ein $H_2O_2$-Überschuß vermieden wird. Die durchgeführten Untersuchungen haben gezeigt, daß normalerweise wenigstens 20 ml $H_2O_2$, gerechnet als 35%ige wäßrige Lösung, je Gramm zu entfernendem $H_2S$ zugesetzt werden sollen. Das $H_2O_2$ wird dabei zweckmäßig in Form einer konzentrierteren, insbesondere einer 20- bis 50%igen Lösung eingesetzt. Die Konzentration kann jedoch auch wesentlich verringert werden, insbesondere wenn in dem geförderten Erdgas bzw. Erdöl selbst relativ wenig Wasser enthalten ist. Bevorzugt werden 50 bis

500 ml $H_2O_2$ je Gramm in der organischen Phase vorliegenden $H_2S$, bezogen auf 35%ige $H_2O_2$-Lösung, verwendet.

Die Erfindung ermöglicht es, auf äußerst einfache Weise $H_2S$ restlos aus der organischen Phase von Erdgas- bzw. Erdölgemischen zu entfernen. Da weder organische Zusätze noch Katalysatoren benötigt werden, ist der Aufwand gering und Schwierigkeiten bei der Zudosierung, wie sie bei Verwendung von Katalysatoren, die dem $H_2O_2$ wegen Zersetzungsgefahr nicht vorher zugesetzt werden können, auftreten, werden vermieden.

Die folgenden Beispiele erläutern die Erfindung weiter:

### Beispiel 1

Bei einem Bohrloch werden pro Stunde 60 bis 65 m³ Erdgas und etwa 3,5 bis 4 m³ wasserhaltiges Erdöl als Gemisch pro Stunde gefördert. Die Temperatur beträgt 30 bis 40°C, der Druck am Bohrlochausgang liegt bei 800 bis 1100 kPa Überdruck.

Der $H_2S$-Gehalt betrug im Mittel 250 mg/m³.

Unter Verwendung einer gegen Überdruck fördernden Pumpe wurden am Bohrlochausgang in die Druckleitung 0,5 l 35%ige wäßrige $H_2O_2$-Lösung pro Stunde eingespritzt. Vom Bohrlochausgang wird das Gas-Öl-Wasser-Gemisch aus dem Bohrloch mit einer Tiefe von ca. 3500 m über eine Druckrohrleitung mit einer inneren lichten Weite von 80 mm in die etwa 2000 m entfernte Aufbereitungsanlage gepumpt. Dort wird in einem Abscheider das Gas von der Flüssigkeit getrennt und in ein Leitungsnetz für Stadtgas eingeführt. Die Flüssigkeit gelangt aus dem Abscheider in einen Trennbehälter, in dem Wasser von Öl geschieden wird. Von dort gelangt das Öl zur Raffinerie, das Wasser in eine Reinigungsanlage.

Am Ende der Druckrohrleitung vor dem Eintritt in die Aufbereitungsanlage wird der $H_2S$-Gehalt mittels eines automatischen, im Handel erhältlichen $H_2S$-Analysators, welcher nach dem colorimetrischen Meßverfahren mit Reagenzpapierband und Bleiazetat als Indikator arbeitet, gemessen.

Die Verweilzeit der geförderten Erdgas-Erdöl-Wasser-Mischung im Druckrohr beträgt etwa 45 Minuten. Bei einem über einen mehrtägigen Zeitraum durchgeführten Betriebsversuch betrug die $H_2S$-Menge am Rohrende 0 mg/m³.

### Beispiel 2

Analog, wie im Beispiel 1 beschrieben, wurde bei einem anderen Bohrloch, bei dem die geförderte Erdgas-Erdöl-Wasser-Mischung im Mittel 165 mg $H_2S$ pro m³ enthielt, $H_2O_2$ zugesetzt. $H_2O_2$-Fördermengen, Zusammensetzung des Fördergutes, Druck, Temperatur und Distanz entsprachen etwa denen von Beispiel 1.

Die $H_2O_2$-Zudosierung in Form von 35%iger wäßriger Lösung wurde so lange gesteigert, bis an der Meßstelle am Ende des Druckrohres ein $H_2S$-Gehalt von 0 bis 5 mg/m³ im Dauerbetrieb gemessen wurde. Die hierbei zudosierte Menge betrug 2,5 l/Stunde.

### Patentansprüche

1. Verfahren zur Beseitigung von $H_2S$ aus Erdgas, Erdöl und deren Gemischen, dadurch gekennzeichnet, daß man das aus der Erde geförderte, wasserhaltige Erdgas, Erdöl oder deren Gemische bei einem Überdruck von mindestens 90 kPa und gegebenenfalls erhöhter Temperatur mit einer wäßrigen $H_2O_2$-Lösung versetzt und in einem Reaktor unter Aufrechterhaltung des erhöhten Drucks unter Mischungsbedingungen mindestens so lange reagieren läßt, bis in der Erdgasphase bzw. Erdölphase die gewünschte Herabsetzung des $H_2S$-Gehaltes eingetreten ist.

2. Verfahren nach Anspruch 1, dadurch gekennzeichnet, daß man einen Überdruck von 500 bis 2000 kPa anwendet.

3. Verfahren nach einem der vorhergehenden Ansprüche, dadurch gekennzeichnet, daß man eine Temperatur zwischen 0 und 60°C anwendet.

4. Verfahren nach einem der vorhergehenden Ansprüche, dadurch gekennzeichnet, daß man einen röhrenförmigen Reaktor anwendet und das Vermischen und die Reaktionsdauer durch die Strömungsgeschwindigkeit im Rohr regelt.

5. Verfahren nach einem der vorhergehenden Ansprüche, dadurch gekennzeichnet, daß man mindestens 20 ml $H_2O_2$ je Gramm $H_2S$ zusetzt.

6. Verfahren nach einem der vorhergehenden Ansprüche, dadurch gekennzeichnet, daß man 20- bis 50%iges $H_2O_2$ zusetzt.

### Claims

1. Process for the removal of $H_2S$ from natural gas, mineral oil or their mixtures, characterised in that one mixes the water-containing natural gas, mineral oil or their mixtures obtained from the ground with an aqueous $H_2O_2$ solution at an increased pressure of at least 90 kPa and optionally elevated temperature and allows to react in a reactor with maintenance of the increased pressure under mixing conditions for so long that the desired reduction of the $H_2S$ content in the natural gas phase or mineral oil phase has taken place.

2. Process according to claim 1, characterised in that one employs an increased pressure of 500 to 2000 kPa.

3. Process according to one of the preceding claims, characterised in that one employs a temperature between 0 and 60°C.

4. Process according to one of the preceding claims, characterised in that one uses a tube-shaped reactor and regulates the mixing and reaction period by the flow rate in the tube.

5. Process according to one of the preceding claims, characterised in that one adds at least 20 ml. $H_2O_2$ per gram of $H_2S$.

6. Process according to one of the preceding claims, characterised in that one adds 20 to 50% $H_2O_2$.


**Revendications**

1. Procédé en vue d'éliminer le $H_2S$ du gaz naturel, du pétrole brut et de leurs mélanges, caractérisé en ce qu'on ajoute une solution aqueuse de $H_2O_2$ au gaz naturel, au pétrole brut ou à leurs mélanges contenant de l'eau et extraits du sol, sous une surpression d'au moins 90 kPa et éventuellement à température élevée, tandis qu'on laisse réagir dans un réacteur en maintenant la pression élevée dans des conditions de mélange au moins jusqu'à ce que la réduction désirée de la teneur en $H_2S$ se soit produite dans la phase de gaz naturel ou la phase de pétrole brut.

2. Procédé suivant la revendication 1, caractérisé en ce qu'on adopte une surpression de 500 à 2000 kPa.

3. Procédé suivant une des revendications précédentes, caractérisé en ce qu'on adopte une température se situant entre 0 et 60°C.

4. Procédé suivant une des revendications précédentes, caractérisé en ce qu'on adopte un réacteur tubulaire, tandis que l'on règle le mélange et le temps de réaction par la vitesse d'écoulement dans le tube.

5. Procédé suivant une des revendications précédentes, caractérisé en ce qu'on ajoute au moins 20 ml de $H_2O_2$ par gramme de $H_2S$.

6. Procédé suivant une des revendications précédentes, caractérisé en ce qu'on ajoute du $H_2O_2$ à 20−50%.